Europäisches Patentamt

European Patent Office

Office européen des brevets

(19)

(11) **EP 1 351 183 A2**

(12) **EUROPEAN PATENT APPLICATION**

(43) Date of publication:
**08.10.2003 Bulletin 2003/41**

(51) Int Cl.$^7$: **G06F 19/00**

(21) Application number: **03251311.1**

(22) Date of filing: **05.03.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **06.03.2002 JP 2002059973**

(71) Applicant: **FUJITSU LIMITED**
**Kawasaki-shi, Kanagawa 211-8588 (JP)**

(72) Inventor: **Sato, Makihiko, c/o Fujitsu Limited**
**Kawasaki-shi, Kanagawa, 211-8588 (JP)**

(74) Representative: **Stebbing, Timothy Charles et al**
**Haseltine Lake & Co.,**
**Imperial House,**
**15-19 Kingsway**
**London WC2B 6UD (GB)**

(54) **Sequence data combining method, apparatus and program**

(57)     Disclosed are a sequence data combining method, apparatus and program, the apparatus (10) capable of creating, from pieces of sequence data that are classified into homology groups, information useful for bio researchers and so on. The sequence data combining apparatus includes a Hidden Markov Model (HMM) creating unit (22) which creates a probability model for each of the homology groups to be processed based on pieces of sequence data in each homology group, an identity value calculation unit (23) which calculates, from each combination of two probability models among the probability models created by said HMM creating unit (22), an identity value which is an index of identity between the two probability models, and a combining unit (24) which specifies similar homology groups based on the identity values calculated by the identity value calculation unit (23), and then creates a homology group by combining the specified homology groups.

FIG. 1

EP 1 351 183 A2

**Description**

The present invention relates to a sequence data combining method, a sequence data combining apparatus, and a sequence data combining program used for re-classifying pieces of sequence data that are classified into homology groups, so as to provide more useful grouping of data, such as DNA or amino acid sequences, for use in biochemical and genetic research.

The present disclosure relates to subject matter contained in Japanese Patent application No. 2002-59973 (filed on March 6, 2002), which is expressly incorporated herein by reference in its entirety.

In the field of biotechnology, research is often carried out by using databases each containing a vast fund of information on DNA sequences and amino acid sequences.

Ordinary databases utilized for biotechnology research contain many pieces of sequence data that are classified into groups called homology groups. There are databases containing several extremely similar, small, homology groups, nevertheless databases in which pieces of sequence data are classified into larger groups (into fewer groups consisting of more pieces of sequence data) are suitable for some research.

It is therefore desirable to provide a sequence data combining method and a sequence data combining apparatus capable of creating, from pieces of sequence data that are classified into homology groups, more useful information (grouping of data) for the bio researchers.

It is also desirable to provide a sequence data combining program capable of directing a computer to combine some pieces of sequence data using the sequence data combining method of the present invention.

According to the present invention, there is provided a sequence data combining method for re-classifying two or more pieces of sequence data that are classified into several homology groups which includes a probability model creating step of creating a probability model for each of the homology groups to be processed based on pieces of sequence data in each homology group; an identity value calculating step of calculating, from each combination of two probability models among the probability models created in the probability model creating step, an identity value which is an index of identity between the two probability models; and a homology group creating step of specifying similar homology groups based on the identity values calculated in the identity value calculation step, and of creating a homology group by combining the specified homology groups.

Namely, the sequence data combining method of the present invention is a method by which more useful information for bio researchers is created, not by checking the identity of pieces of sequence data, but by combining some existing homology groups. Consequently, using this sequence data combining method, useful information for research can be prepared more rapidly.

When implementing the sequence data combining method of the present invention, it is possible in the probability model creating step to create an HMM (Hidden Markov Model) as the probability model, or in the identity value calculating step in which the identity value is calculated to use dynamic programming techniques. Further, it is possible to adapt the homology group creating step, to further involve creating a probability model for the created homology group.

A sequence data combining apparatus according to the present invention for re-classifying two or more pieces of sequence data that are classified into several homology groups includes a probability model creating unit for creating a probability model for each of the homology groups based on pieces of sequence data in each homology group; an identity value calculating unit for calculating, from each combination of two probability models among the probability models created by the probability model creating unit, an identity value which is an index of identity between the two probability models; and a homology group creating unit for specifying similar homology groups based on the identity values calculated by the identity value calculating unit, and of creating a homology group by combining the specified homology groups.

That is, the sequence data combining apparatus according to the present invention is configured so as to be able to perform the sequence data combining method according to the present invention. Consequently, when using this sequence data combining apparatus of the present invention, it is possible to prepare useful information more rapidly.

According to the present invention there is also provided a sequence data combining program for causing a computer to execute a process for re-classifying two or more pieces of sequence data that are classified into several homology groups, said process comprising: a probability model creating step of creating a probability model for each of the homology groups to be processed based on pieces of sequence data in each homology group; an identity value calculating step of calculating, from each combination of two probability models among the probability models created in said probability model creating step, an identity value which is an index of identity between the two probability models; and a homology group creating step of specifying similar homology groups based on the identity values calculated in said identity value calculating step, and of creating a homology group by combining the specified homology groups.

Consequently, when using the program of the present invention, it is possible to prepare useful information more rapidly. Hence according to the method, apparatus and program of the present invention, complex data sequences can be combined and reclassified into more appropriate groups quicker than by conventional methods.

These and other features and advantages of the present invention and its embodiments will now be described with reference, by way of example only, to the accompanying drawings, wherein:

FIG. 1 is a functional block diagram of a sequence data combining apparatus of an embodiment of the present invention;

FIG. 2 is a diagram illustrating an HMM created by the sequence data combining apparatus of this embodiment;

FIG. 3 is a diagram illustrating pairwise alignment using dynamic programming methods;

FIG. 4 is a diagram illustrating calculation results calculated by the identity value calculating unit;

FIG. 5 is a diagram illustrating the homology group (HG) information-$\alpha$ from which HMM-$\alpha$ in FIG. 4 is created;

FIG. 6 is a diagram illustrating the homology group information-$\beta$ from which HMM-$\beta$ in FIG. 4 is created;

FIG. 7 is a diagram illustrating the homology group information-$\gamma$ from which HMM-$\gamma$ in FIG. 4 is created;

FIG. 8A is a diagram illustrating the relationship between HMM—$\alpha$ and HMM—$\beta$;

FIG. 8B is a diagram illustrating the relationship between HMM—$\alpha$ and HMM—$\gamma$;

FIG. 8C is a diagram illustrating the relationship between HMM-$\beta$ and HMM-$\gamma$; and

FIG. 9 is a diagram illustrating the homology group information the combining unit creates.

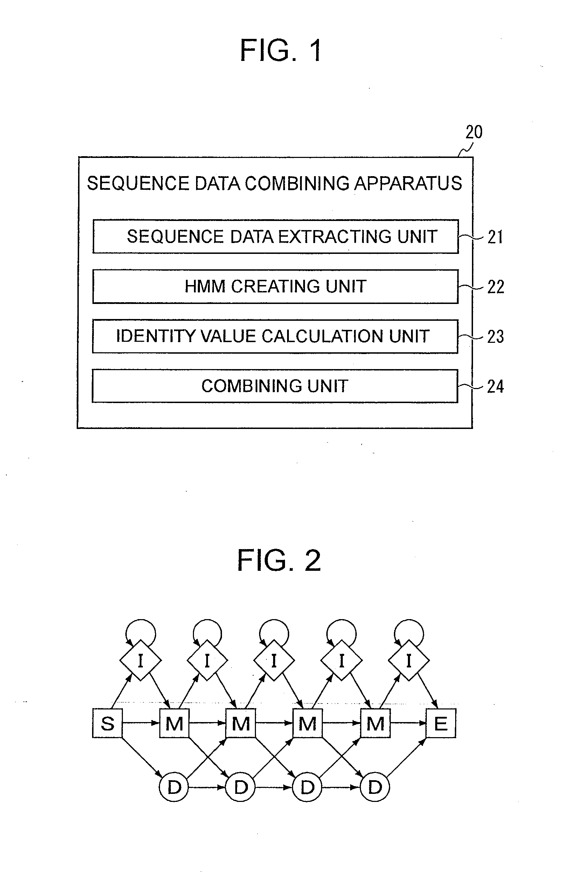

FIG. 1 is a functional block diagram of a sequence data combining apparatus 10 of an embodiment of the present invention.

The sequence data combining apparatus 10 of this embodiment is realized as a device wherein a sequence data combining program is installed on a relatively high-performance computer. The sequence data combining apparatus 10 functions, as shown in this figure, as a device that includes a sequence data extracting unit 21, an HMM creating unit 22, an identity value calculation unit 23, and a combining unit 24 (homology group creating unit).

<SEQUENCE DATA EXTRACTING UNIT>

The sequence data extracting unit 21 is a unit for extracting, from a database of gene sequences and/or amino acid sequences, some pieces of homology group (HG) information (collection of pieces of sequence data that are classified into a homology group) that meet a retrieval condition inputted by an operator, and for storing the extracted information into an auxiliary storage (not shown in FIG. 1) in the sequence data combining apparatus 10. The sequence data extracting unit 21 starts the above processing when the operator performs operations including inputting of the retrieval condition to an input device of the sequence data combining apparatus 10.

Each piece of homology group information that the sequence data extracting unit 21 extracts is a collection of pieces of multiple-aligned sequence data. Multiple alignment is an operation (process) for obtaining from three or more sequences new sequences in which elements are lined up in the most similar order by inserting gaps into appropriate locations of the original sequences. In the following paragraphs, the term "alignment" is also used to describe the result of the alignment processing.

<HMM CREATING UNIT>

The HMM creating unit 22 is a unit for creating an HMM (Hidden Markov Model) from each piece of the homology group information extracted by the sequence data extracting unit 21.

As shown in FIG. 2, the HMM is a probability model that comprises M nodes, I nodes, D nodes, S nodes and E nodes made to correlate with each other via a transition probability (shown by arrows in the figure).

The M nodes and I nodes included in this HMM are nodes each expressing the state of a certain element of a sequence (or a sequence alignment). The M node is the node to which emission probability of symbols (in HMMs expressing a base sequence there are four types of emission probability for four types of symbols referred to as A, G, C and T, and in HMMs expressing amino acid sequences, there are twenty types of emission probability) and the probability of a transition to several other nodes (M nodes, I nodes and D nodes) is assigned. The I node is the node to which emission probabilities for a plurality of symbols and several transition probabilities to several other nodes are assigned. However, the probability of a transition between I nodes is made to correspond at one I node rather than being the probability of a transition to another I node.

The D node is the dummy node to which no emission probability is assigned. Only the probabilities of transitions to several nodes are assigned to the D node. The S node is the node expressing the start state (initial state) of this HMM, and only the probabilities of transitions to several other nodes are assigned to this S node. The E node is the node expressing the end state (final state) of this HMM, and only emission probabilities are assigned to this E node.

Processing which the HMM creating unit 22 performs to create a HMM follows the usual method for creating a HMM. Therefore, the explanation of the procedure of creating the HMM by the HMM creating unit 22 will be omitted.

<IDENTITY VALUE CALCULATION UNIT>

The identity value calculation unit 23 (FIG. 1) is a unit for calculating, from each couple (pair) of HMMs (combination of two HMMs) among all HMMs created by the HMM creation unit 22, an identity value that is an index of identity (similarity) of the couple of HMMs.

Arithmetic processing executed by the HMM creating unit 22 is a variation of arithmetic processing employing dynamic programming techniques carried out in the related art for pairwise alignment.

Therefore, first, a description of the arithmetic processing employing dynamic programming techniques will be given.

Put in simple terms, pairwise alignment is an operation (usually performed by computer processing) for obtaining two sequences in which elements are lined up in a most similar (coincident) order by inserting gaps into appropriate locations of two sequences that are to be processed.

An outline of pairwise alignment using dynamic programming techniques is now described giving an example of the case where pairwise alignment is carried out on two sequences (character strings) referred to as "AIMS" and "AMOS".

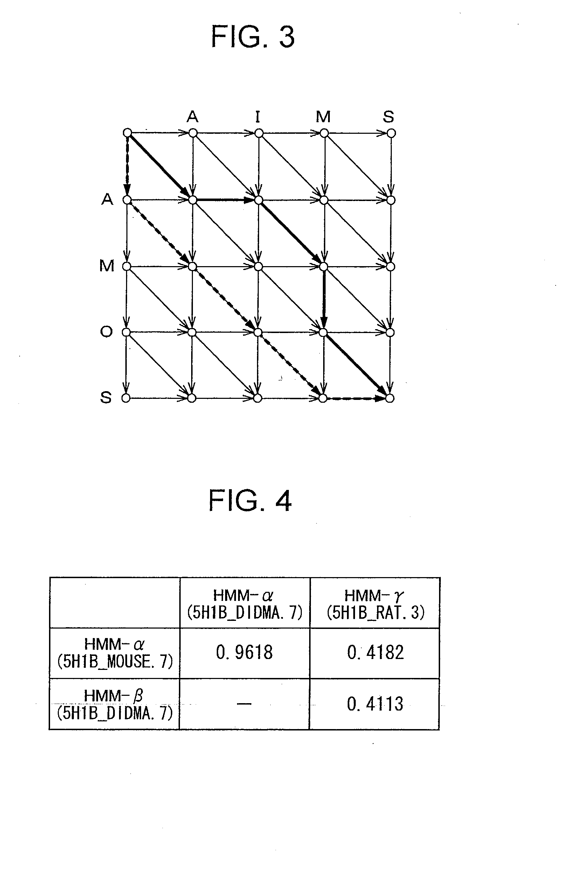

In this case, as shown schematically in FIG. 3, the existence of a matrix containing 5x5 nodes (circles) is assumed, with specific elements of one sequence (referred to in the following as a "first sequence" and in the drawings as "AIMS") to be aligned being made to correspond to a group of nodes lined up in the vertical direction, and specific elements of a second sequence (referred to in the following as a "second sequence" and in the drawings as "AMOS") to be aligned being made to correspond with nodes that are lined up horizontally.

When obtaining pairwise alignment, each migration path along the direction of the arrows from the node at the upper left end of the matrix to the node at the lower right end can be understood as one alignment (one alignment result for two series).

Specifically, with respect to the first sequence, movement along the arrows towards the right can be understood to be an operation of outputting elements (characters) made to correspond to nodes after movement as elements of alignment results, and with regards to the second sequence, movement to the right along the direction of the arrows can be understood to be an operation of outputting gaps as elements of alignment results. Further, with regard to both the first and second sequences, movement at an incline along the direction of the arrows can be understood as an operation for outputting elements (characters) made to correspond to nodes after movement as elements of alignment results. Regarding the first sequence, movement downwards along the direction of the arrows can be understood as an operation of outputting gaps as elements of alignment results, while regarding the second sequence, this movement can be understood as an operation of outputting elements (characters) made to correspond to nodes after movement as elements of alignment results.

Namely, in this figure, the path shown by the dotted line can be understood as showing "-AIMS" and "AMOS-", while the path shown by the thick lined arrows can be understood as showing "AIM-S" and "A-MOS".

If the most similar items are specified from all of the alignment results that this matrix expresses, then the optimal alignment can be obtained. However, with regard to all of the alignment results, it is desired to evaluate the extent to which the two sequences are similar after alignment, and obtaining the alignment that is the objective is time consuming.

In order to shorten this period of time, the following equation 1 (a recursive formula for i, j) is used for obtaining an evaluation point (evaluation value) for each path.

$$V_{i,j} = \begin{cases} w_{i,j} + V_{i-1,j-1} \\ d + V_{i,j-1} \\ d + V_{i-1,j} \end{cases} \quad \cdots \text{eq.1}$$

In this equation 1, $V_{i,j}$ is an evaluation point (evaluation value) for a path to a node making a first sequence element #i and a second sequence element #j correspond. {} is a function which outputs maximum element, and d is an evaluation point for a deficiency of corresponding elements referred to as "gap penalty" or "gap cost". Further, $w_{i,j}$ is an evaluation point relating to identity (similarity) between the first sequence element #i and the second sequence element #j. Note that a value (one of two preset values) corresponding to whether or not both elements coincide is used as $w_{i,j}$ when a base sequence is taken as a subject and a value read out from a table storing w values for each combination of two amino acids is used when an amino acid sequence is taken as the subject.

The calculation of equation 1 is then carried out for each node by increasing i, j sequentially while obtaining pairwise alignment using dynamic programming techniques. The optimum alignment is then obtained by storing which of the paths traced was the most appropriate (a plurality is also possible), and then, after completion of all the calculations, tracing the optimum path back (trace back) in reverse from the lower right end.

Thus, the pairwise alignment employing dynamic programming techniques can therefore be completed at high speed, because carried out is a process in which every calculation of a V value increases paths (those without optimum alignment) for which final evaluation points are not calculated (a process in which, with the max function {}, paths for two of three types of path capable of reaching this node are taken to be paths for which calculation of the final evaluation point is not carried out).

Next, a description is given of the operation of the identity value calculation unit 23.

The identity value calculation unit 23 is for subjecting the HMM to processing by the same theory as for the processing carried out in order to obtain pairwise alignment.

Specifically, in the identity value calculation processing executed by the identity value calculation unit 23, a matrix comprising (imax + 1) x (jmax + 1) nodes where emission probability vectors for ith M nodes relating to HMM#0 (one of the two HMMs to be subjected to sequence data combining) are made to correspond to emission probability vectors for jth M nodes relating to HMM #1 (the other of the two HMMs to be subjected to sequence data combining) is assumed. Here, HMM#0 is one of the two HMMs to be subjected to sequence data combining, and HMM #1 is the other of the two HMMs, and imax is the number of M nodes of the HMM#0, and jmax is the number of M nodes of the HMM#1.

In the identity value calculation processing, evaluation values $V_{i, j}$, which are evaluation values for nodes (i, j) of the evaluation matrix, are calculated using equation 2 described in the following.

$$V_{i,j} = \left[ \begin{array}{c} \dfrac{S(M_i, M_j) + V_{i-1, j-1}}{L} \\ \dfrac{d + V_{i, j-1}}{L'} \\ \dfrac{d + V_{i-1, j}}{L''} \end{array} \right] \quad \cdots \text{ eq.2}$$

In equation 2, d is a so-called gap cost (gap penalty), and L, L' and L'' are the number of nodes that are passed through to reach node (i, j). The introduction of L, L' and L'' is so that an evaluation value for a path with a large number of gaps inserted is a relatively small value.

Further, $M_i$ is an emission probability vector for the ith M node of HMM#0, and $M_j$ is an emission probability vector for the jth M node of HMM#1. $S(M_i, M_j)$ is a function for obtaining an identity constituted by numerical information exhibiting this identity from the emission probability vector $M_i$ and the emission probability vector $M_j$. Any function may be employed as $S(M_i, M_j)$ providing that a maximum value (for example, "1") is taken when $M_i$ and $M_j$ are the same, and a minimum value (for example, "0") is taken when $M_i$ and $M_j$ are completely different (when $M_i$ and $M_j$ are orthogonal). Namely, as shown in FIG. 4, the cosine cos(è) of the angle è between the vectors $M_i$ and $M_j$ or the cosine squared $\cos^2$ (è) of the angle è can be used as S(M_i, Mj), but the identity value calculation unit 23 of this embodiment employs the cosine squared cos2(è) of the angle è as $S(M_i, M_j)$.

<COMBINING UNIT>

The combining unit 24 is a unit for combining pieces of HG information extracted by the identity value calculation unit 23, based on the calculation results of the identity value calculation unit 23.

The combining unit 24 starts operation when the identity value calculation unit 23 finishes the calculation processing. The combining unit 24 tries to specify (select) every couple of HMMs the identity value of which is lower than a predetermined identity threshold value. When the combining unit 24 has specified one or more couples of HMMs, it starts combining processing for combining the specified couples of HMMs.

Hereinafter, the combining processing executed by the combining unit 24 is described giving an example of the case where the identity values calculated by the identity value calculation unit 23 are those shown in FIG. 4 and the identity threshold value is 0.9.

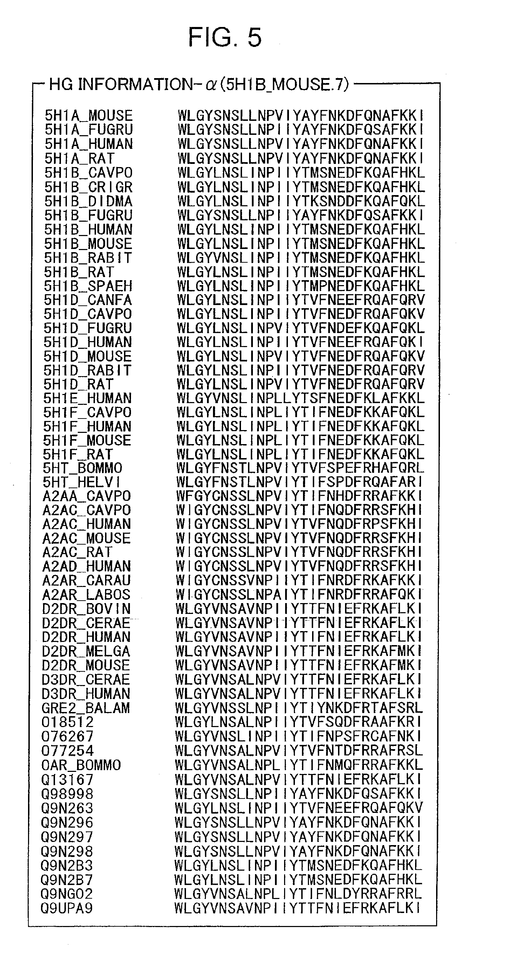

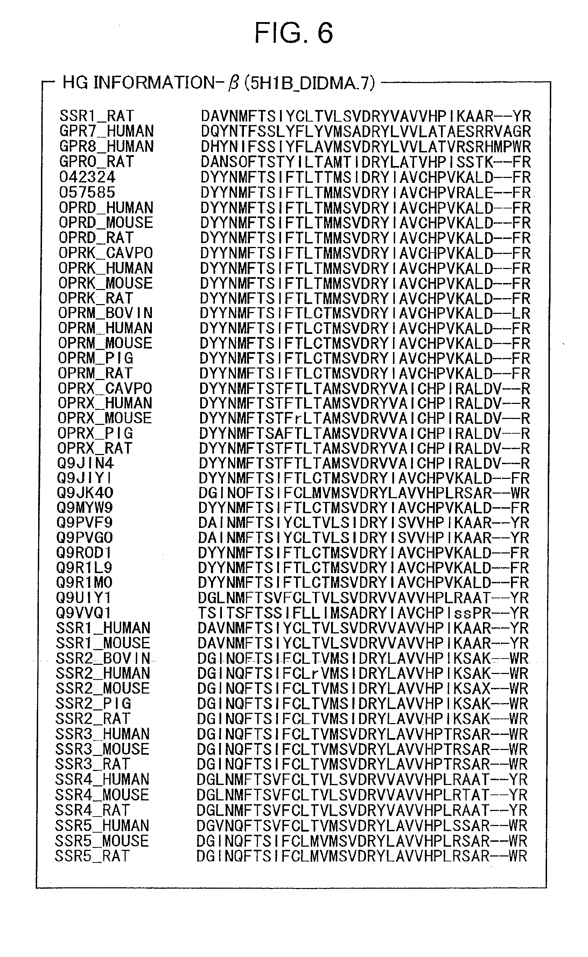

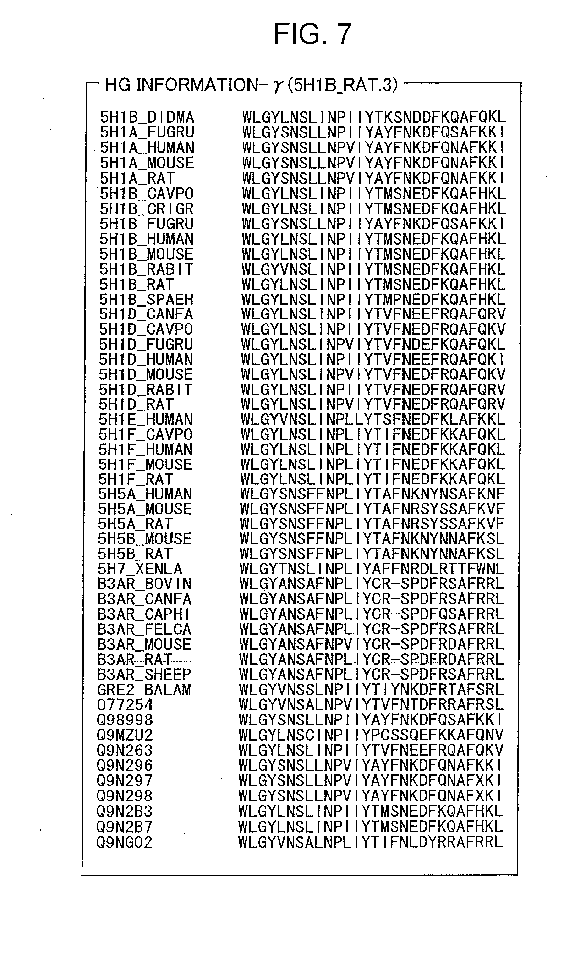

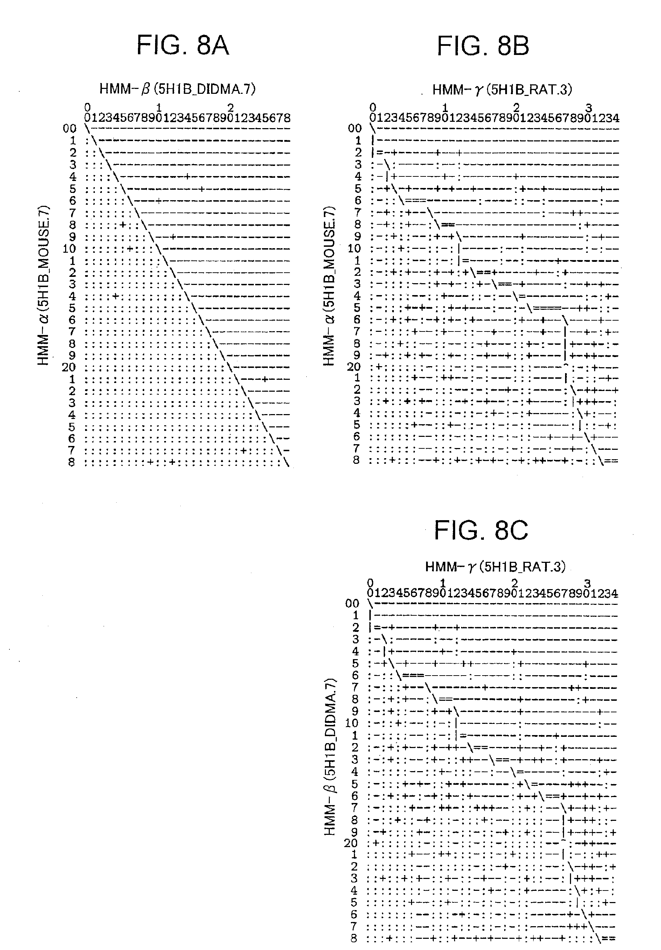

Here, the HMM-α, HMM-β, and HMM-γ in FIG. 4 are HMMs created by the HMM creation unit 22 from HG information-α (5H1B_MOUSE.7) shown in FIG. 5, HG information-β (5H1B_DIDMA.7) shown in FIG. 6, and HG information-γ (SH1B_RAT.3) shown in FIG. 7, respectively. And the HMM-α and HMM-β, the HMM-α and HMM-γ , the HMM-β and HMM-γ are HMMs having the relationships shown in FIGs 8A-8C, which show the back trace results of the identity calculation processing by the identity value calculation unit 23, respectively. Incidentally, in FIG. 8A-8C, portions described by "\", "|", "=" are back traced portions, and the portions described by "\", "|", "=" are portions connected by diagonal, up, and sideways (left), respectively. Further, portions described by each of the symbols "+", ":" and "-" are non-back traced portions and show portions connected from diagonal, up, sideways (left) (i.e. connected by diagonal, down, right).

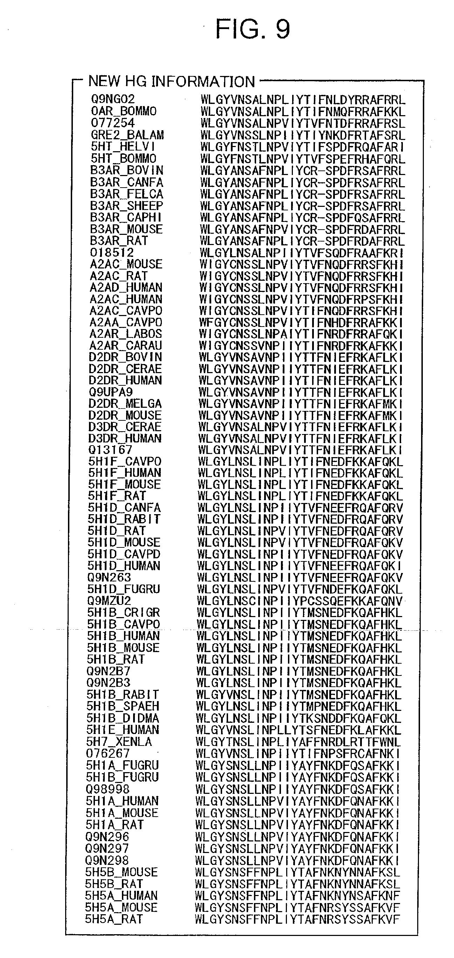

In this case, only the identity value of the couple (pair) of HMM—α and HMM-β is higher than the identity threshold value. Therefore, the combining unit 24 extracts from the HG information-α and the HG information-β all sequence data but without duplication. The combining unit 24, thereafter, executes multiple alignment processing on the extracted sequence data, thereby creating new HG information as shown in FIG. 9, and stores the created HG information into the auxiliary storage. Further, the combining unit 24 creates a HMM from the created HG information and stores the created HMM into the auxiliary storage and then terminates the processing.

As described in detail above, the sequence data combining apparatus 10 of this embodiment is configured so as to be able to retrieve similar HG information from pieces of HG information, and combine the retrieved two or more pieces of HG information. In other words, the sequence data combining apparatus 10 is configured so as to create some pieces of new HG information which are more useful for biochemical, genetic etc. research, not by checking the identity of pieces of sequence data, but by combining some pieces of existing HG information. Furthermore, the sequence data combining apparatus 10 has the ability to create a HMM of the created HG information. Therefore, if this sequence data combining apparatus 10 is used, it is possible to prepare useful information on gene sequence and the likes for the bio researchers rapidly.

<Modification>

Various modifications are possible for the sequence data combining apparatus 10 described above. For example, the sequence data combining apparatus 10 is configured so as to calculate $V_{i,j}$ using eq. 2. In other words, the sequence data combining apparatus 10 is configured so as to calculate the identity value of the two HMMs considering only the emission probabilities assigned to M nodes. However, the sequence data combining apparatus 10 can be modified so as to calculate $V_{i,\,j}$ using eq. 3 instead of eq. 1.

$$V_{i,j} = \begin{bmatrix} \dfrac{S(T_i,T_j) \cdot S(M_i,M_j) + V_{i-1,\,j-1}}{L} \\ \dfrac{d + V_{i,\,j-1}}{L'} \\ \dfrac{d + V_{i-1,\,j}}{L''} \end{bmatrix} \quad \cdots \text{eq.3}$$

In eq. 3, Ti is a transition probability vector for the ith M node of HMM#0, and Tj is an emission probability vector for the jth M node of HMM#1. $S(T_i, T_j)$ is the identity between the two transition probability vectors ($S(T_i, T_j)$ is the cosine squared of the angle made by the two vectors).

The sequence data combining apparatus 10 can be also modified so as to calculate $V_{i,j}$ using equations 4 to 7 instead of eq. 1.

$$V_{i,j} = \begin{bmatrix} \dfrac{Sim_{i,\,j} + V_{i-1,\,j-1}}{L} \\ \dfrac{\max(d,\,D1_{i,\,j-1}) + V_{i,\,j-1}}{L'} \\ \dfrac{\max(d,\,D2_{i-1,\,j}) + V_{i-1,\,j}}{L''} \end{bmatrix} \quad \cdots \text{eq.4}$$

$$Sim_{i,j} = \frac{Tm_i \cdot Tm_j \cdot S(M_i, M_j) + Ti_i \cdot Ti_j \cdot S(I_i, I_j) + Td_i \cdot Td_j}{|T_i| \cdot |T_j|} \qquad \cdots eq.5$$

$$D1_{i,j} = \frac{Ti_i \cdot Tm_j \cdot S(I_i, M_j) + Tm_i \cdot Td_j}{|T_i| \cdot |T_j|} \qquad \cdots eq.6$$

$$D2_{i,j} = \frac{Ti_j \cdot Tm_i \cdot S(I_j, M_i) + Tm_j \cdot Td_i}{|T_i| \cdot |T_j|} \qquad \cdots eq.7$$

In these equations, $Tm_i$, $Ti_i$ and $Td_i$ are the probability of a transition to an M node, the probability of a transition to an I node, and the probability of a transition to a D node, respectively, with regard to the ith M node of the HMM#0. Tmj, Tij and Tdj are the probability of a transition to an M node, the probability of a transition to an I node, and the probability of a transition to a D node, respectively, with regard to the jth M node of the HMM#1. $I_i$ is an emission probability vector for the ith node of HMM#0, and $I_j$ is an emission probability vector for the jth I node of HMM#1.

Moreover, the sequence data combining apparatus 10 can be configured so that the combining unit 24 operates as follows.

The combining unit 24, when the operation of the identity value calculation unit 23 ends, displays a standby screen on the display device. Here, the standby screen is a screen where frequency distribution information on the identity values and the current threshold value are shown. In other words, the standby screen is a screen which allows the operator to know how many pieces of HG information will be combined by the current threshold value.

After displaying the standby screen, the combining unit 24 goes into a standby state and waits for input of a change instruction indicating a change of the identity threshold value, an execution instruction indicating to start combining processing and so on.

When the change instruction is input, the combining unit 24 displays a screen for prompting the operator to input a new identity threshold value, and goes into a state where it waits for input of the new identity threshold value. When the new identity threshold value is input, the combining unit 24 stores the inputted identity threshold value. Thereafter, the combining unit 24 displays the standby screen where the inputted identity threshold value is shown on the display device, and goes back into the standby state.

When the execution instruction is input, the combining unit 24 specifies each couple of HMMs whose identity values are higher than the identity threshold value. And, the combining unit 24, when it has specified at least one couple of HMMs, executes the combining processing of combining pieces of HMM information related to the specified one or more couple of HMMs.

In short, the sequence data combining apparatus 10 can be configured so as to operate interactively.

Further, the sequence data combining apparatus 10 is a device where the sequence data combining program is installed on a computer. It is possible to realize the sequence data combining apparatus 10 having an IC that operates as the identity value calculation unit 23 and so on. The technology employed in the sequence data combining apparatus 10 may also be applied to probability models other than HMMs. Moreover, a portable record medium (CD-ROM and MO, etc.) containing the sequence data combining program may be distributed (sold) to a person who wants it.

The method, apparatus and program of the present invention provide fast and efficient data sorting and grouping for large quantities of complex data.

**Claims**

1.    A sequence data combining method for re-classifying two or more pieces of sequence data that are classified into several homology groups, including:

a probability model creating step of creating a probability model for each of the homology groups to be processed based on pieces of sequence data in each homology group;
an identity value calculating step of calculating, from each combination of two probability models among the probability models created in said probability model creating step, an identity value which is an index of identity between the two probability models; and
a homology group creating step of specifying similar homology groups based on the identity values calculated in said identity value calculation step, and of creating a homology group by combining the specified homology groups.

2. The sequence data combining method according to claim 1, wherein the probability model created in said probability model creating step is a Hidden Markov Model.

3. The sequence data combining method according to claim 1 or 2, wherein the identity value calculating step is a step of calculating the identity value using dynamic programming techniques.

4. The sequence data combining method according to claim 1, 2, or 3, wherein the homology group creating step involves creating a probability model for the created homology group.

5. A sequence data combining apparatus for re-classifying two or more pieces of sequence data that are classified into several homology groups, including:

a probability model creating unit for creating a probability model for each of the homology groups to be processed based on pieces of sequence data in each homology group;
an identity value calculating unit for calculating, from each combination of two probability models among the probability models created by said probability model creating unit, an identity value which is an index of identity between the two probability models; and
a homology group creating unit for specifying similar homology groups based on the identity values calculated by said identity value calculating unit, and of creating a homology group by combining the specified homology groups.

6. The sequence data combining apparatus according to claim 5, wherein the probability model created by said probability model creating unit is a Hidden Markov Model.

7. The sequence data combining apparatus according to claim 5 or 6, wherein the identity value calculating unit calculates the identity value using dynamic programming techniques.

8. A sequence data combining program for causing a computer to execute a process for re-classifying two or more pieces of sequence data that are classified into several homology groups, said process comprising:

a probability model creating step of creating a probability model for each of the homology groups to be processed based on pieces of sequence data in each homology group;
an identity value calculating step of calculating, from each combination of two probability models among the probability models created in said probability model creating step, an identity value which is an index of identity between the two probability models; and
a homology group creating step of specifying similar homology groups based on the identity values calculated in said identity value calculating step, and of creating a homology group by combining the specified homology groups.

9. The sequence data combining program according to claim 8, wherein the probability model created in said probability model creating step is a Hidden Markov Model.

10. The sequence data combining program according to claim 8 or 9, wherein the identity value calculated in the identity value calculating step is calculated using dynamic programming techniques.

11. An apparatus for re-classifying two or more pieces of data that are classified into several homology groups, including:

probability model creating means for creating a probability model for each of the homology groups to be processed based on pieces of data in each homology group;

identity value calculating means for calculating, from each combination of two probability models among the probability models created by said probability model creating means, an identity value which is an index of identity between the two probability models; and

homology group creating means for specifying similar homology groups based on the identity values calculated by said identity value calculating means, and for creating a homology group by combining the specified homology groups.

# FIG. 1

```
┌─────────────────────────────────────────────┐ 20
│ SEQUENCE DATA COMBINING APPARATUS            │
│  ┌─────────────────────────────────────┐     │
│  │ SEQUENCE DATA EXTRACTING UNIT       │─── 21│
│  └─────────────────────────────────────┘     │
│  ┌─────────────────────────────────────┐     │
│  │ HMM CREATING UNIT                   │─── 22│
│  └─────────────────────────────────────┘     │
│  ┌─────────────────────────────────────┐     │
│  │ IDENTITY VALUE CALCULATION UNIT     │─── 23│
│  └─────────────────────────────────────┘     │
│  ┌─────────────────────────────────────┐     │
│  │ COMBINING UNIT                      │─── 24│
│  └─────────────────────────────────────┘     │
└─────────────────────────────────────────────┘
```

# FIG. 2

## FIG. 3

## FIG. 4

|  | HMM-$\alpha$ (5H1B_DIDMA. 7) | HMM-$\gamma$ (5H1B_RAT. 3) |
|---|---|---|
| HMM-$\alpha$ (5H1B_MOUSE. 7) | 0.9618 | 0.4182 |
| HMM-$\beta$ (5H1B_DIDMA. 7) | — | 0.4113 |

# FIG. 5

```
┌─ HG INFORMATION- α (5H1B_MOUSE.7) ──────────────┐
│                                                  │
│  5H1A_MOUSE      WLGYSNSLLNPVIYAYFNKDFQNAFKKI    │
│  5H1A_FUGRU      WLGYSNSLLNPIIYAYFNKDFQSAFKKI    │
│  5H1A_HUMAN      WLGYSNSLLNPVIYAYFNKDFQNAFKKI    │
│  5H1A_RAT        WLGYSNSLLNPVIYAYFNKDFQNAFKKI    │
│  5H1B_CAVPO      WLGYLNSLINPIIYTMSNEDFKQAFHKL    │
│  5H1B_CRIGR      WLGYLNSLINPIIYTMSNEDFKQAFHKL    │
│  5H1B_DIDMA      WLGYLNSLINPIIYTKSNDDFKQAFQKL    │
│  5H1B_FUGRU      WLGYSNSLLNPIIYAYFNKDFQSAFKKI    │
│  5H1B_HUMAN      WLGYLNSLINPIIYTMSNEDFKQAFHKL    │
│  5H1B_MOUSE      WLGYLNSLINPIIYTMSNEDFKQAFHKL    │
│  5H1B_RABIT      WLGYVNSLINPIIYTMSNEDFKQAFHKL    │
│  5H1B_RAT        WLGYLNSLINPIIYTMSNEDFKQAFHKL    │
│  5H1B_SPAEH      WLGYLNSLINPIIYTMPNEDFKQAFHKL    │
│  5H1D_CANFA      WLGYLNSLINPIIYTVFNEEFRQAFQRV    │
│  5H1D_CAVPO      WLGYLNSLINPIIYTVFNEDFRQAFQKV    │
│  5H1D_FUGRU      WLGYLNSLINPVIYTVFNDEFKQAFQKL    │
│  5H1D_HUMAN      WLGYLNSLINPIIYTVFNEEFRQAFQKI    │
│  5H1D_MOUSE      WLGYLNSLINPVIYTVFNEDFRQAFQKV    │
│  5H1D_RABIT      WLGYLNSLINPIIYTVFNEDFRQAFQRV    │
│  5H1D_RAT        WLGYLNSLINPVIYTVFNEDFRQAFQRV    │
│  5H1E_HUMAN      WLGYVNSLINPLLYTSFNEDFKLAFKKL    │
│  5H1F_CAVPO      WLGYLNSLINPLIYTIFNEDFKKAFQKL    │
│  5H1F_HUMAN      WLGYLNSLINPLIYTIFNEDFKKAFQKL    │
│  5H1F_MOUSE      WLGYLNSLINPLIYTIFNEDFKKAFQKL    │
│  5H1F_RAT        WLGYLNSLINPLIYTIFNEDFKKAFQKL    │
│  5HT_BOMMO       WLGYFNSTLNPVIYTVFSPEFRHAFQRL    │
│  5HT_HELVI       WLGYFNSTLNPVIYTIFSPDFRQAFARI    │
│  A2AA_CAVPO      WFGYCNSSLNPVIYTIFNHDFRRAFKKI    │
│  A2AC_CAVPO      WIGYCNSSLNPVIYTIFNQDFRRSFKHI    │
│  A2AC_HUMAN      WIGYCNSSLNPVIYTVFNQDFRPSFKHI    │
│  A2AC_MOUSE      WIGYCNSSLNPVIYTVFNQDFRRSFKHI    │
│  A2AC_RAT        WIGYCNSSLNPVIYTVFNQDFRRSFKHI    │
│  A2AD_HUMAN      WIGYCNSSLNPVIYTVFNQDFRRSFKHI    │
│  A2AR_CARAU      WIGYCNSSVNPIIYTIFNRDFRKAFKKI    │
│  A2AR_LABOS      WIGYCNSSLNPAIYTIFNRDFRRAFQKI    │
│  D2DR_BOVIN      WLGYVNSAVNPIIYTTFNIEFRKAFLKI    │
│  D2DR_CERAE      WLGYVNSAVNPIIYTTFNIEFRKAFLKI    │
│  D2DR_HUMAN      WLGYVNSAVNPIIYTIFNIEFRKAFLKI    │
│  D2DR_MELGA      WLGYVNSAVNPIIYTTFNIEFRKAFMKI    │
│  D2DR_MOUSE      WLGYVNSAVNPIIYTTFNIEFRKAFMKI    │
│  D3DR_CERAE      WLGYVNSALNPVIYTTFNIEFRKAFLKI    │
│  D3DR_HUMAN      WLGYVNSALNPVIYTTFNIEFRKAFLKI    │
│  GRE2_BALAM      WLGYVNSSLNPIIYTIYNKDFRTAFSRL    │
│  O18512          WLGYLNSALNPIIYTVFSQDFRAAFKRI    │
│  O76267          WLGYVNSLINPIIYTIFNPSFRCAFNKI    │
│  O77254          WLGYVNSALNPVIYTVFNTDFRRAFRSL    │
│  OAR_BOMMO       WLGYVNSALNPLIYTIFNMQFRRAFKKL    │
│  Q13167          WLGYVNSALNPVIYTTFNIEFRKAFLKI    │
│  Q98998          WLGYSNSLLNPIIYAYFNKDFQSAFKKI    │
│  Q9N263          WLGYLNSLINPIIYTVFNEEFRQAFQKV    │
│  Q9N296          WLGYSNSLLNPVIYAYFNKDFQNAFKKI    │
│  Q9N297          WLGYSNSLLNPVIYAYFNKDFQNAFKKI    │
│  Q9N298          WLGYSNSLLNPVIYAYFNKDFQNAFKKI    │
│  Q9N2B3          WLGYLNSLINPIIYTMSNEDFKQAFHKL    │
│  Q9N2B7          WLGYLNSLINPIIYTMSNEDFKQAFHKL    │
│  Q9NGO2          WLGYVNSALNPLIYTIFNLDYRRAFRRL    │
│  Q9UPA9          WLGYVNSAVNPIIYTTFNIEFRKAFLKI    │
│                                                  │
└──────────────────────────────────────────────────┘
```

# FIG. 6

┌─ HG INFORMATION-β (5H1B_DIDMA.7) ─────────────────────

```
SSR1_RAT       DAVNMFTSIYCLTVLSVDRYVAVVHPIKAAR--YR
GPR7_HUMAN     DQYNTFSSLYFLYVMSADRYLVVLATAESRRVAGR
GPR8_HUMAN     DHYNIFSSIYFLAVMSVDRYLVVLATVRSRHMPWR
GPRO_RAT       DANSOFTSTYILTAMTIDRYLATVHPISSTK---FR
042324         DYYNMFTSIFTLTTMSIDRYIAVCHPVKALD--FR
057585         DYYNMFTSIFTLTMMSVDRYIAVCHPVRALE--FR
OPRD_HUMAN     DYYNMFTSIFTLTMMSVDRYIAVCHPVKALD--FR
OPRD_MOUSE     DYYNMFTSIFTLTMMSVDRYIAVCHPVKALD--FR
OPRD_RAT       DYYNMFTSIFTLTMMSVDRYIAVCHPVKALD--FR
OPRK_CAVPO     DYYNMFTSIFTLTMMSVDRYIAVCHPVKALD--FR
OPRK_HUMAN     DYYNMFTSIFTLTMMSVDRYIAVCHPVKALD--FR
OPRK_MOUSE     DYYNMFTSIFTLTMMSVDRYIAVCHPVKALD--FR
OPRK_RAT       DYYNMFTSIFTLTMMSVDRYIAVCHPVKALD--FR
OPRM_BOVIN     DYYNMFTSIFTLCTMSVDRYIAVCHPVKALD--LR
OPRM_HUMAN     DYYNMFTSIFTLCTMSVDRYIAVCHPVKALD--FR
OPRM_MOUSE     DYYNMFTSIFTLCTMSVDRYIAVCHPVKALD--FR
OPRM_PIG       DYYNMFTSIFTLCTMSVDRYIAVCHPVKALD--FR
OPRM_RAT       DYYNMFTSIFTLCTMSVDRYIAVCHPVKALD--FR
OPRX_CAVPO     DYYNMFTSTFTLTAMSVDRYVAICHPIRALDV--R
OPRX_HUMAN     DYYNMFTSTFTLTAMSVDRYVAICHPIRALDV--R
OPRX_MOUSE     DYYNMFTSTFrLTAMSVDRVVAICHPIRALDV--R
OPRX_PIG       DYYNMFTSAFTLTAMSVDRVVAICHPIRALDV--R
OPRX_RAT       DYYNMFTSTFTLTAMSVDRYVAICHPIRALDV--R
Q9JIN4         DYYNMFTSTFTLTAMSVDRVVAICHPIRALDV---R
Q9JIYI         DYYNMFTSIFTLCTMSVDRYIAVCHPVKALD--FR
Q9JK40         DGINOFTSIFCLMVMSVDRYLAVVHPLRSAR--WR
Q9MYW9         DYYNMFTSIFTLCTMSVDRYIAVCHPVKALD--FR
Q9PVF9         DAINMFTSIYCLTVLSIDRYISVVHPIKAAR--YR
Q9PVGO         DAINMFTSIYCLTVLSIDRYISVVHPIKAAR--YR
Q9ROD1         DYYNMFTSIFTLCTMSVDRYIAVCHPVKALD--FR
Q9R1L9         DYYNMFTSIFTLCTMSVDRYIAVCHPVKALD--FR
Q9R1MO         DYYNMFTSIFTLCTMSVDRYIAVCHPVKALD--FR
Q9UIY1         DGLNMFTSVFCLTVLSVDRVVAVVHPLRAAT--YR
Q9VVQ1         TSITSFTSSIFLLIMSADRYIAVCHPIssPR--YR
SSR1_HUMAN     DAVNMFTSIYCLTVLSVDRVVAVVHPIKAAR--YR
SSR1_MOUSE     DAVNMFTSIYCLTVLSVDRVVAVVHPIKAAR--YR
SSR2_BOVIN     DGINOFTSIFCLTVMSIDRYLAVVHPIKSAK--WR
SSR2_HUMAN     DGINQFTSIFCLrVMSIDRYLAVVHPIKSAK--WR
SSR2_MOUSE     DGINQFTSIFCLTVMSIDRYLAVVHPIKSAX--WR
SSR2_PIG       DGINQFTSIFCLTVMSIDRYLAVVHPIKSAK--WR
SSR2_RAT       DGINQFTSIFCLTVMSIDRYLAVVHPIKSAK--WR
SSR3_HUMAN     DGINQFTSIFCLTVMSVDRYLAVVHPTRSAR--WR
SSR3_MOUSE     DGINQFTSIFCLTVMSVDRYLAVVHPTRSAR--WR
SSR3_RAT       DGINQFTSIFCLTVMSVDRYLAVVHPTRSAR--WR
SSR4_HUMAN     DGLNMFTSVFCLTVLSVDRVVAVVHPLRAAT--YR
SSR4_MOUSE     DGLNMFTSVFCLTVLSVDRVVAVVHPLRTAT--YR
SSR4_RAT       DGLNMFTSVFCLTVLSVDRYVAVVHPLRAAT--YR
SSR5_HUMAN     DGVNQFTSVFCLTVMSVDRYLAVVHPLSSAR--WR
SSR5_MOUSE     DGINQFTSIFCLMVMSVDRYLAVVHPLRSAR--WR
SSR5_RAT       DGINQFTSIFCLMVMSVDRYLAVVHPLRSAR--WR
```

# FIG. 7

HG INFORMATION-$\gamma$ (5H1B_RAT.3)

| | |
|---|---|
| 5H1B_DIDMA | WLGYLNSLINPIIYTKSNDDFKQAFQKL |
| 5H1A_FUGRU | WLGYSNSLLNPIIYAYFNKDFQSAFKKI |
| 5H1A_HUMAN | WLGYSNSLLNPVIYAYFNKDFQNAFKKI |
| 5H1A_MOUSE | WLGYSNSLLNPVIYAYFNKDFQNAFKKI |
| 5H1A_RAT | WLGYSNSLLNPVIYAYFNKDFQNAFKKI |
| 5H1B_CAVPO | WLGYLNSLINPIIYTMSNEDFKQAFHKL |
| 5H1B_CRIGR | WLGYLNSLINPIIYTMSNEDFKQAFHKL |
| 5H1B_FUGRU | WLGYSNSLLNPIIYAYFNKDFQSAFKKI |
| 5H1B_HUMAN | WLGYLNSLINPIIYTMSNEDFKQAFHKL |
| 5H1B_MOUSE | WLGYLNSLINPIIYTMSNEDFKQAFHKL |
| 5H1B_RABIT | WLGYVNSLINPIIYTMSNEDFKQAFHKL |
| 5H1B_RAT | WLGYLNSLINPIIYTMSNEDFKQAFHKL |
| 5H1B_SPAEH | WLGYLNSLINPIIYTMPNEDFKQAFHKL |
| 5H1D_CANFA | WLGYLNSLINPIIYTVFNEEFRQAFQRV |
| 5H1D_CAVPO | WLGYLNSLINPIIYTVFNEDFRQAFQKV |
| 5H1D_FUGRU | WLGYLNSLINPVIYTVFNDEFKQAFQKL |
| 5H1D_HUMAN | WLGYLNSLINPIIYTVFNEEFRQAFQKI |
| 5H1D_MOUSE | WLGYLNSLINPVIYTVFNEDFRQAFQKV |
| 5H1D_RABIT | WLGYLNSLINPIIYTVFNEDFRQAFQRV |
| 5H1D_RAT | WLGYLNSLINPVIYTVFNEDFRQAFQRV |
| 5H1E_HUMAN | WLGYVNSLINPLLYTSFNEDFKLAFKKL |
| 5H1F_CAVPO | WLGYLNSLINPLIYTIFNEDFKKAFQKL |
| 5H1F_HUMAN | WLGYLNSLINPLIYTIFNEDFKKAFQKL |
| 5H1F_MOUSE | WLGYLNSLINPLIYTIFNEDFKKAFQKL |
| 5H1F_RAT | WLGYLNSLINPLIYTIFNEDFKKAFQKL |
| 5H5A_HUMAN | WLGYSNSFFNPLIYTAFNKNYNSAFKNF |
| 5H5A_MOUSE | WLGYSNSFFNPLIYTAFNRSYSSAFKVF |
| 5H5A_RAT | WLGYSNSFFNPLIYTAFNRSYSSAFKVF |
| 5H5B_MOUSE | WLGYSNSFFNPLIYTAFNKNYNNAFKSL |
| 5H5B_RAT | WLGYSNSFFNPLIYTAFNKNYNNAFKSL |
| 5H7_XENLA | WLGYTNSLINPLIYAFFNRDLRTTFWNL |
| B3AR_BOVIN | WLGYANSAFNPLIYCR-SPDFRSAFRRL |
| B3AR_CANFA | WLGYANSAFNPLIYCR-SPDFRSAFRRL |
| B3AR_CAPH1 | WLGYANSAFNPLIYCR-SPDFQSAFRRL |
| B3AR_FELCA | WLGYANSAFNPLIYCR-SPDFRSAFRRL |
| B3AR_MOUSE | WLGYANSAFNPVIYCR-SPDFRDAFRRL |
| B3AR_RAT | WLGYANSAFNPLIYCR-SPDFRDAFRRL |
| B3AR_SHEEP | WLGYANSAFNPLIYCR-SPDFRSAFRRL |
| GRE2_BALAM | WLGYVNSSLNPIIYTIYNKDFRTAFSRL |
| O77254 | WLGYVNSALNPVIYTVFNTDFRRAFRSL |
| Q98998 | WLGYSNSLLNPIIYAYFNKDFQSAFKKI |
| Q9MZU2 | WLGYLNSCINPIIYPCSSQEFKKAFQNV |
| Q9N263 | WLGYLNSLINPIIYTVFNEEFRQAFQKV |
| Q9N296 | WLGYSNSLLNPVIYAYFNKDFQNAFKKI |
| Q9N297 | WLGYSNSLLNPVIYAYFNKDFQNAFXKI |
| Q9N298 | WLGYSNSLLNPVIYAYFNKDFQNAFXKI |
| Q9N2B3 | WLGYLNSLINPIIYTMSNEDFKQAFHKL |
| Q9N2B7 | WLGYLNSLINPIIYTMSNEDFKQAFHKL |
| Q9NG02 | WLGYVNSALNPLIYTIFNLDYRRAFRRL |

## FIG. 8A

HMM-$\beta$ (5H1B_DIDMA.7)

HMM-$\alpha$ (5H1B_MOUSE.7)

## FIG. 8B

HMM-$\gamma$ (5H1B_RAT.3)

HMM-$\alpha$ (5H1B_MOUSE.7)

## FIG. 8C

HMM-$\gamma$ (5H1B_RAT.3)

HMM-$\beta$ (5H1B_DIDMA.7)

# FIG. 9

```
┌─ NEW HG INFORMATION ──────────────────────────────────┐
│ Q9NG02          WLGYVNSALNPLIYTIFNLDYRRAFRRL           │
│ OAR_BOMMO       WLGYVNSALNPLIYTIFNMQFRRAFKKL           │
│ O77254          WLGYVNSALNPVIYTVFNTDFRRAFRSL           │
│ GRE2_BALAM      WLGYVNSSLNPIIYTIYNKDFRTAFSRL           │
│ 5HT_HELVI       WLGYFNSTLNPVIYTIFSPDFRQAFARI           │
│ 5HT_BOMMO       WLGYFNSTLNPVIYTVFSPEFRHAFQRL           │
│ B3AR_BOVIN      WLGYANSAFNPLIYCR-SPDFRSAFRRL           │
│ B3AR_CANFA      WLGYANSAFNPLIYCR-SPDFRSAFRRL           │
│ B3AR_FELCA      WLGYANSAFNPLIYCR-SPDFRSAFRRL           │
│ B3AR_SHEEP      WLGYANSAFNPLIYCR-SPDFRSAFRRL           │
│ B3AR_CAPHI      WLGYANSAFNPLIYCR-SPDFQSAFRRL           │
│ B3AR_MOUSE      WLGYANSAFNPVIYCR-SPDFRDAFRRL           │
│ B3AR_RAT        WLGYANSAFNPLIYCR-SPDFRDAFRRL           │
│ O18512          WLGYLNSALNPIIYTVFSQDFRAAFKRI           │
│ A2AC_MOUSE      WIGYCNSSLNPVIYTVFNQDFRRSFKHI           │
│ A2AC_RAT        WIGYCNSSLNPVIYTVFNQDFRRSFKHI           │
│ A2AD_HUMAN      WIGYCNSSLNPVIYTVFNQDFRRSFKHI           │
│ A2AC_HUMAN      WIGYCNSSLNPVIYTVFNQDFRPSFKHI           │
│ A2AC_CAVPO      WIGYCNSSLNPVIYTIFNQDFRRSFKHI           │
│ A2AA_CAVPO      WFGYCNSSLNPVIYTIFNHDFRRAFKKI           │
│ A2AR_LABOS      WIGYCNSSLNPAIYTIFNRDFRRAFQKI           │
│ A2AR_CARAU      WIGYCNSSVNPIIYTIFNRDFRKAFKKI           │
│ D2DR_BOVIN      WLGYVNSAVNPIIYTTFNIEFRKAFLKI           │
│ D2DR_CERAE      WLGYVNSAVNPIIYTTFNIEFRKAFLKI           │
│ D2DR_HUMAN      WLGYVNSAVNPIIYTIFNIEFRKAFLKI           │
│ Q9UPA9          WLGYVNSAVNPIIYTTFNIEFRKAFLKI           │
│ D2DR_MELGA      WLGYVNSAVNPIIYTTFNIEFRKAFMKI           │
│ D2DR_MOUSE      WLGYVNSAVNPIIYTTFNIEFRKAFMKI           │
│ D3DR_CERAE      WLGYVNSALNPVIYTTFNIEFRKAFLKI           │
│ D3DR_HUMAN      WLGYVNSALNPVIYTTFNIEFRKAFLKI           │
│ Q13167          WLGYVNSALNPVIYTTFNIEFRKAFLKI           │
│ 5H1F_CAVPO      WLGYLNSLINPLIYTIFNEDFKKAFQKL           │
│ 5H1F_HUMAN      WLGYLNSLINPLIYTIFNEDFKKAFQKL           │
│ 5H1F_MOUSE      WLGYLNSLINPLIYTIFNEDFKKAFQKL           │
│ 5H1F_RAT        WLGYLNSLINPLIYTIFNEDFKKAFQKL           │
│ 5H1D_CANFA      WLGYLNSLINPIIYTVFNEEFRQAFQRV           │
│ 5H1D_RABIT      WLGYLNSLINPIIYTVFNEDFRQAFQRV           │
│ 5H1D_RAT        WLGYLNSLINPVIYTVFNEDFRQAFQRV           │
│ 5H1D_MOUSE      WLGYLNSLINPVIYTVFNEDFRQAFQKV           │
│ 5H1D_CAVPD      WLGYLNSLINPIIYTVFNEDFRQAFQKV           │
│ 5H1D_HUMAN      WLGYLNSLINPIIYTVFNEEFRQAFQKI           │
│ Q9N263          WLGYLNSLINPIIYTVFNEEFRQAFQKV           │
│ 5H1D_FUGRU      WLGYLNSLINPVIYTVFNDEFKQAFQKL           │
│ Q9MZU2          WLGYLNSCINPIIYPCSSQEFKKAFQNV           │
│ 5H1B_CRIGR      WLGYLNSLINPIIYTMSNEDFKQAFHKL           │
│ 5H1B_CAVPO      WLGYLNSLINPIIYTMSNEDFKQAFHKL           │
│ 5H1B_HUMAN      WLGYLNSLINPIIYTMSNEDFKQAFHKL           │
│ 5H1B_MOUSE      WLGYLNSLINPIIYTMSNEDFKQAFHKL           │
│ 5H1B_RAT        WLGYLNSLINPIIYTMSNEDFKQAFHKL           │
│ Q9N2B7          WLGYLNSLINPIIYTMSNEDFKQAFHKL           │
│ Q9N2B3          WLGYLNSLINPIIYTMSNEDFKQAFHKL           │
│ 5H1B_RABIT      WLGYVNSLINPIIYTMSNEDFKQAFHKL           │
│ 5H1B_SPAEH      WLGYLNSLINPIIYTMPNEDFKQAFHKL           │
│ 5H1B_DIDMA      WLGYLNSLINPIIYTKSNDDFKQAFQKL           │
│ 5H1E_HUMAN      WLGYVNSLINPLLYTSFNEDFKLAFKKL           │
│ 5H7_XENLA       WLGYTNSLINPLIYAFFNRDLRTTFWNL           │
│ O76267          WLGYVNSLINPIIYTIFNPSFRCAFNKI           │
│ 5H1A_FUGRU      WLGYSNSLLNPIIYAYFNKDFQSAFKKI           │
│ 5H1B_FUGRU      WLGYSNSLLNPIIYAYFNKDFQSAFKKI           │
│ Q98998          WLGYSNSLLNPIIYAYFNKDFQSAFKKI           │
│ 5H1A_HUMAN      WLGYSNSLLNPVIYAYFNKDFQNAFKKI           │
│ 5H1A_MOUSE      WLGYSNSLLNPVIYAYFNKDFQNAFKKI           │
│ 5H1A_RAT        WLGYSNSLLNPVIYAYFNKDFQNAFKKI           │
│ Q9N296          WLGYSNSLLNPVIYAYFNKDFQNAFKKI           │
│ Q9N297          WLGYSNSLLNPVIYAYFNKDFQNAFKKI           │
│ Q9N298          WLGYSNSLLNPVIYAYFNKDFQNAFKKI           │
│ 5H5B_MOUSE      WLGYSNSFFNPLIYTAFNKNYNNAFKSL           │
│ 5H5B_RAT        WLGYSNSFFNPLIYTAFNKNYNNAFKSL           │
│ 5H5A_HUMAN      WLGYSNSFFNPLIYTAFNKNYNSAFKNF           │
│ 5H5A_MOUSE      WLGYSNSFFNPLIYTAFNRSYSSAFKVF           │
│ 5H5A_RAT        WLGYSNSFFNPLIYTAFNRSYSSAFKVF           │
└────────────────────────────────────────────────────────┘
```